(19) Europäisches Patentamt
European Patent Office
Office européen des brevets

(11) EP 2 317 977 B1

(12) **EUROPEAN PATENT SPECIFICATION**

(45) Date of publication and mention of the grant of the patent:
**23.07.2014 Bulletin 2014/30**

(21) Application number: **09792023.5**

(22) Date of filing: **28.08.2009**

(51) Int Cl.:
***A61K 9/00*** *(2006.01)* ***A61K 47/02*** *(2006.01)*
***A61K 47/10*** *(2006.01)* ***A61K 47/12*** *(2006.01)*
***A61K 47/18*** *(2006.01)* ***A61K 9/127*** *(2006.01)*

(86) International application number:
**PCT/US2009/055276**

(87) International publication number:
**WO 2010/027907 (11.03.2010 Gazette 2010/10)**

(54) **PHARMACEUTICAL COMPOSITION HAVING RELATIVELY LOW IONIC STRENGTH**

PHARMAZEUTISCHE ZUSAMMENSETZUNG MIT RELATIV GERINGER IONENSTÄRKE

COMPOSITION PHARMACEUTIQUE PRÉSENTANT UNE FORCE IONIQUE RELATIVEMENT FAIBLE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.09.2008 US 93900 P**

(43) Date of publication of application:
**11.05.2011 Bulletin 2011/19**

(73) Proprietor: **Alcon Research, Ltd.**
**Fort Worth, TX 76134-2099 (US)**

(72) Inventors:
- **GHOSH, Malay**
**Fort Worth**
**Texas 76109 (US)**
- **CHOWHAN, Masood, A.**
**Arlington**
**Texas 76016 (US)**

(74) Representative: **Teipel, Stephan et al**
**Lederer & Keller**
**Patentanwälte Partnerschaft mbB**
**Unsöldstrasse 2**
**80538 München (DE)**

(56) References cited:
**WO-A1-2008/144494**
**WO-A2-01/70230**
**US-A- 5 985 310**
**US-A1- 2006 160 818**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 317 977 B1

## Description

### Technical Field of the Invention

The present invention is related to pharmaceutical compositions having relatively low ionic strengths that result in improved preservation for the compositions. More specifically the present invention relates to ophthalmic, otic or nasal pharmaceutical compositions (e.g., multi-dose aqueous ophthalmic compositions) that are formulated to include a preservative, a therapeutic agent or both and a relatively low concentration of ionic solutes.

### Background of the Invention

The present invention is directed to pharmaceutical compositions, particularly ophthalmic compositions, formulated so as to have sufficient antimicrobial activity to satisfy the preservation efficacy requirements of the United States Pharmacopeia ("USP") and analogous guidelines in other countries. The ability to achieve preservation is based on a unique combination of one or more anti-microbial agents or preservatives and a relatively low amount of ionic solutes.

Many pharmaceutical compositions are required to be sterile (i.e., substantially free of bacteria, fungi and other pathogenic microorganisms). Examples of such compositions include: solutions and suspensions that are injected into the bodies of humans or other mammals; creams, lotions, solutions or other preparations that are topically applied to wounds, abrasions, burns, rashes, surgical incisions, or other conditions where the skin is not intact; and various types of compositions that are applied either directly to the eye (e.g., artificial tears, irrigating solutions, and drug products), or are applied to devices that will come into contact with the eye (e.g., contact lenses).

The foregoing types of compositions can be manufactured under sterile conditions via procedures that are well known to those skilled in the art. However, once the packaging for a product is opened, such that the composition contained therein is exposed to the atmosphere and other sources of potential microbial contamination (e.g., the hands of a human patient), the sterility of the product may be compromised. Such products are typically utilized multiple times by the patient, and are therefore frequently referred to as being of a "multi-dose" nature.

Due to the frequent, repeated exposure of multi-dose products to the risk of microbial contamination, it is necessary to employ a means for preventing such contamination from occurring. The means employed may be: (i) a chemical agent that prevents the proliferation of microbes in a composition, which is referred to herein as an "antimicrobial preservative"; or (ii) a packaging system that prevents or reduces the risk of microbes reaching a pharmaceutical composition within a container.

Prior multi-dose compositions have generally contained one or more antimicrobial preservatives in order to prevent the proliferation of bacteria, fungi and other microbes. Many different preservatives are presently known and making a choice as to which preservative or preservatives are desirable for a particular composition can be difficult and can involve multiple competing factors.

As one example, it is generally desirable to employ lower concentrations of preservative to minimize any toxicity that might be caused by the preservative[s], however, higher concentrations of preservatives are typically desirable for achieving greater preservation efficacy. As an additional or alternative example, certain preservatives exhibit more or less efficacy depending upon the conditions (e.g., pH, tonicity or the like) or ingredients present within a particular pharmaceutical composition.

In view of these competing factors and/or difficulties, it is desirable to provide pharmaceutical compositions, particularly ophthalmic, otic and/or nasal pharmaceutical compositions (e.g., aqueous solutions, suspensions or gels) that promote the efficacy of one or more preservatives within those compositions.

### Summary of the Invention

Accordingly, there is disclosed a pharmaceutical composition that includes an antimicrobial preservative and water. The ant-microbial preservative is typically significantly affected by ionic strength and is selected from sorbate, non-polymeric diquaternary ammonium compound or a combination thereof. The composition is typically aqueous and the ionic strength of the composition is less than 0.08 $mol*L^{-1}$. When present, the non-polymeric diquaternary ammonium compound is one that typically includes a phosphate group. The compositions of the present invention are also quite ideal as multi-dose compositions.

It is preferable for the composition to include a therapeutic agent. In one embodiment, the therapeutic agent is one that is more soluble and possibly quite substantially more soluble at lower pH levels. In such an embodiment the pH of the composition is typically less than 7.0, more typically less than 6.0, even more typically less than 5.5 and still more typically less than 5.2. One example of such a therapeutic agent is tandospirone.

The invention relates to a pharmaceutical composition, comprising anti-microbial preservative that is significantly affected by ionic strength wherein the preservative is selected from sorbate or a non-polymeric diquaternary ammonium

phosphate compound and water;
wherein, when the preservative is a non-polymeric diquaternary ammonium phosphate compound, the composition has an ionic strength that is less than 0.08 mol*$L^{-1}$; and
wherein, when the non-polymeric diquaternary ammonium phosphate compound is sodium coco diammonium chloride phosphate, the concentration of the sodium coco diammonium chloride phosphate in the composition is 0.0025 to 0.004 w/v%;
or, when the preservative is sorbate, the concentration of sorbate in the composition is from 0.01 to 0.2 w/v% and the ionic strength of the composition is less than 0.07 mol*$L^{-1}$.

## Detailed Description of the Invention

The present invention is predicated upon the provision of preserved pharmaceutical compositions having one or more anti-microbial preservatives where the preservation efficacy of the one or more preservatives is significantly affected by ionic strength. As such, the efficacy of the one or more preservatives is maintained or enhanced by formulating the compositions to have low ionic strengths. The compositions will also typically include one or more therapeutic agents or ingredients and/or conditions within the compositions that interfere with preservation efficacy of more conventional preservatives.

Unless otherwise stated, percentages of ingredients are expressed in terms of weight volume percents (w/v%).

The pharmaceutical compositions of the present invention are formulated so as to maintain an overall low ionic strength. This is accomplished by maintaining the number and/or concentration of ionic ingredients or solutes at relatively low levels. Examples of such ionic ingredients include ionic salts such as sodium chloride (NaCl). Further examples of ionic solutes include potassium chloride, magnesium chloride and calcium chloride.

As used herein, ionic strength is defined as a characteristic of the pharmaceutical composition (preferably an aqueous composition) that is expressed as the average electrostatic interactions among ions of the composition. The ionic strength is half of the total, which obtained by multiplying the molality (the amount of substance per unit mass of solvent) of each ion by its valence squared. The ionic strength, *I*, of the composition is a function of the concentrations of all ions present in a solution and is expressed by the following equation:

$$I = \frac{1}{2} \sum C_i z_i^2$$

where $C_i$ is the molar concentration of an ion (mol*$L^{-1}$) within the composition, z is the charge number of that ion, and the sum is taken over all ions (i) in the composition. For an electrolyte such as sodium chloride, the ionic strength is equal to half of the concentration since the charge number of sodium chloride is one, but for $MgSO_4$ the ionic strength is a half of four times its concentration since the charge number of $MgSO_4$ is two. Therefore, contribution of multivalent ions to the ionic strength in the composition is more profound compared to monovalent species.

The ingredients of the composition can be divided into ionic and nonionic components. Ionic components are those that dissociate into ionic form in the composition and nonionic components are those that do not dissociate. Then, the ionic strength can be determined in accordance with the equations provided above. The relationship between preservative concentration and ionic strength in the composition has been found to be an important factor in determining preservation efficacy. As the concentration of ionic strength affected preservative increases, the ability of the compositions to meet USP preservative efficacy requirements will typically increase, however, the relatively high concentrations of ionic strength affected preservative needed to maintain such efficacy are typically undesirable.

Generally, it is preferable for the ionic strength of the compositions of the present invention to be less than 0.20, more typically less than 0.12 and even more particularly less than 0.08 mol*$L^{-1}$. However, for particular compositions, more specific ionic strength guidelines are provided herein.

Typically the entirety, at least a portion or at least a substantial portion (e.g. at least 50%, 70%, 90% or more) of the anti-microbial preservative is formed of one or more preservatives where the preservation efficacy of those preservative[s] is significantly affected by the ionic strength of the composition. As used herein, the term "significantly affected by ionic strength" as it applies to preservative[s] in compositions of the present invention means that a raise of 0.4 w/v% sodium chloride in a composition that includes such preservative[s] and passes USP or Ph. Eur. B as described below will cause the composition to fail those same standards in at least one and more preferably at least two categories selected from S. Aureus, P. Aerugin., E. Coli, C. Albican and A. Niger at 24 hours. For the present invention, particularly preferred preservatives that are significantly affected by ionic strength include sorbate, diquaternary ammonium compound or a combination thereof.

The diquaternary ammonium compound is typically non-polymeric and amphoteric in nature. Moreover, in preferred embodiments, the diquaternary ammonium compounds includes a phosphate group and contains phosphate functionality. Particularly preferred are diquaternary ammonium compounds of formula (I). Formula (I) is as follows:

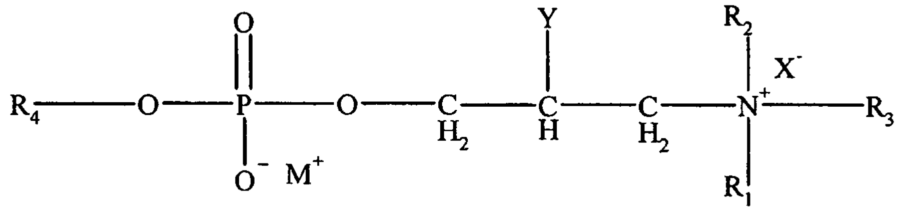

(I)

wherein:

$R_1$ and $R_3$ are ($C_1$-$C_6$)-alkyl;

$R_2$ is selected from the group consisting of hydrogen and ($C_1$-$C_{16}$)-alkyl optionally substituted by NHC(=O)-$(CH_2)_{10}CH_3$ or NHC(=O)-$(CH_2)_{12}CH_3$;

$R_4$ is selected from the group consisting of hydrogen and $CH_2CH(Y)CH_2N^+R_1R_2R_3X^-$, wherein $R_1$, $R_2$, and $R_3$, are as defined above;

X is halo;

Y is selected from the group consisting of OH, O-($C_1$-$C_{10}$)-alkyl and O-($C_1$-$C_{10}$)-alkenyl; and

M is selected from the group consisting of sodium and potassium.

In the foregoing definitions of $R_1$, $R_2$, $R_3$, $R_4$, X, Y and M substituents, and throughout, the following terms unless otherwise indicated, shall be understood to have the following meanings:

The term "alkenyl" includes straight or branched chain hydrocarbon groups having 1 to 30 carbon atoms with at least one carbon-carbon double bond, the chain being optionally interrupted by one or more heteroatoms. The chain hydrogens may be substituted with other groups, such as, halo, $-CF_3$, $-NO_2$, $-NH_2$, -CN, - $OCH_3$, $-C_6H_5$, -O-$C_6H_5$O-alkyl, -O-$C_6H_5$O-alkenyl, p-NHC(=O)-$C_6H_5$-NHC(=O)-$CH_3$, -CH=NH, -NHC(=O)-Ph and -SH. Preferred straight or branched alkenyl groups include allyl, ethenyl, propenyl, butenyl pentenyl, hexenyl, heptenyl, octenyl, nonenyl, decenyl, undecenyl, dodecenyl, tridecenyl, tetradecenyl, pentadecenyl or hexadecenyl.

The term "alkyl" includes straight or branched chain aliphatic hydrocarbon groups that are saturated and have 1 to 30 carbon atoms. The alkyl groups may be interrupted by one or more heteroatoms, such as oxygen, nitrogen, or sulfur, and may be substituted with other groups, such as, halo, $-CF_3$, $-NO_2$, $NH_2$, -CN, - $OCH_3$, $-C_6H_5$, -O-$C_6H_5$O-alkyl, -O-$C_6H_5$O-alkenyl, p-NHC(=O)-$C_6H_5$-NHC(=O)-$CH_3$, -CH=NH, -NHC(=O)-Ph and -SH. Preferred straight or branched alkyl groups include methyl, ethyl, propyl, isopropyl, butyl, t-butyl, sec-butyl, pentyl, hexyl, heptyl, octyl, nonyl, decyl, undecyl and dodecyl, tridecyl, tetradecyl, pentadecyl or hexadecyl.

The term "halo" means an element of the halogen family. Preferred halo moieties include fluorine, chlorine, bromine or iodine.

The unique molecular arrangement of the synthetic diquaternary ammonium compound (i.e., wherein a phosphate group is linked to a quaternary ammonium functionality via a substituted-propenyl group, and the quaternary ammonium functionality is further linked to at least one long hydrocarbon chain) are what make them highly water soluble. In particular, the length of the hydrocarbon chain and the ionic functional groups are important factors to consider for maintaining solubility and efficacy of the molecules for the uses described herein.

The preferred compounds of formula (I) are those wherein $R_1$ and $R_3$ are methyl; $R_2$ is selected from the group consisting of $(CH_2)_{11}CH_3$, $(CH_2)_3$-NHC(=O)-$(CH_2)_{10}CH_3$ and $(CH_2)_3$-NHC(=O)-$(CH_2)_{12}CH_3$; $R_4$ is $CH_2CH(Y)CH_2N^+R_1R_2R_3X^-$, wherein $R_1$, $R_2$, and $R_3$, are as defined above; X is chloro; Y is OH; and M is sodium. The most preferred compounds are identified in the following table:

| SUBSTITUENT | COMPOUND NO. 1 | COMPOUND NO. 2 | COMPOUND NO. 3 |
|---|---|---|---|
| $R_1$ | $-CH_3$ | $-CH_3$ | $-CH_3$ |
| $R_2$ | $-(CH_2)_{11}CH_3$ | $-(CH_2)_3-NHC(=O)-(CH_2)_{10}CH_3$ | $-(CH_2)_3-NHC(=O)-(CH_2)_{12}CH_3$ |
| $R_3$ | $-CH_3$ | $-CH_3$ | $-CH_3$ |
| $R_4$ | 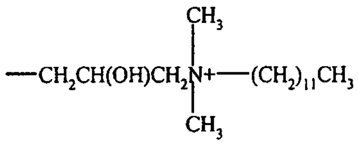 | 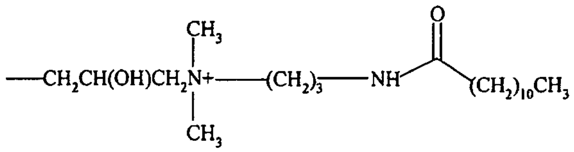 | 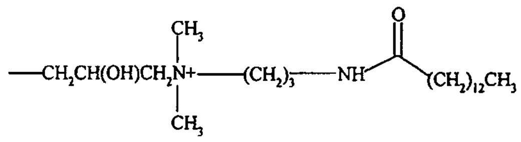 |
| $X^-$ | $Cl^-$ | $Cl^-$ | $Cl^-$ |
| Y | -OH | -OH | -OH |
| $M^+$ | $Na^+$ | $Na^+$ | $Na^+$ |

The compounds of formula (I) can be synthesized in accordance with known procedures (see for example, U.S. Patent Nos. 5,286,719; 5,648,348 and 5,650,402) and/or purchased from commercial sources, such as Uniquema (Cowick Hall, Snaith, Goole East Yorkshire, DN149AA). Compounds of formula (I) are further discussed in U.S. Patent Application 12/122,197, filed May 16, 2008, entitled "Phospholipid Compositions for Contact Lens Care and Preservation of Pharmaceutical Compositions", which is incorporated herein by reference for all purposes. One particularly preferred compound of formula (I) is sodium coco diammonium chloride phosphate (SCDCP).

As suggested, the other preferred preservative that is significantly affected by ionic strength is sorbate. As used herein, the term "sorbate" shall refer to sorbic acid, salts of sorbic acid, sorbate derivatives and other pharmaceutically acceptable sorbates, or combinations thereof. Most suitable are: sorbic acid, sodium sorbate, potassium sorbate, calcium sorbate, magnesium sorbate, manganese sorbate, and other such sorbate salts.

Typically, the pharmaceutical composition includes at least 0.0001 %, more typically at least 0.0009 % and even more typically at least 0.003 % preservative[s] that are significantly affected by ionic strength. The pharmaceutical composition also typically includes less than 1.0 %, more typically less than 0.1 % and even more typically less than 0.03 % preservative[s] that are significantly affected by ionic strength. These concentrations are particularly preferred for circumstances in which the preservatives that are significantly affected by ionic strength are selected from sorbate, synthetic diquaternary ammonium (e.g., non-polymeric diquaternary ammonium phosphate compound) or a combination thereof.

Highly preferred ranges of concentrations of particular preservatives relative to ionic strengths have also been developed. When the concentration of sorbate is from about 0.01 to about 0.2 w/v%, the ionic strength of the composition is preferably less than about 0.07 mole/L and preferably from about 0.01 mole/L to about 0.05 mole/L. When the concentration of non-polymeric diquaternary ammonium phosphate compound (e.g., SCDCP) is from about 0.01 to 0.1 %, the ionic strength of the composition is preferably less than about 0.165 mole/L and preferably from about 0.1 mole/L to about 0.15 mole/L. When the concentration of non-polymeric diquaternary ammonium phosphate compound (e.g., SCDCP) is from about 0.005 to 0.01 w/v%, the ionic strength of the composition is preferably less than about 0.125 mole/L and preferably from about 0.07 mole/L to about 0.1 mole/L. When the concentration of non-polymeric diquaternary ammonium phosphate compound (e.g., SCDCP) is from about 0.0025 to 0.004 w/v%, the ionic strength of the composition is preferably less than about 0.09 mole/L and preferably from about 0.01 mole/L to about 0.07 mole/L. Of course, unless otherwise specifically stated, these ranges are merely guidelines and higher or lower ranges may be used within the scope of the present invention.

Generally, the compositions of the present invention (i.e., the compositions including at least one preservative significantly affected by ionic strength), can be used for various applications such as contact lens care compositions or pharmaceutical compositions. Advantageously, however, the compositions of the present invention are quite desirable as ophthalmic, otic or nasal pharmaceutical compositions (particularly ophthalmic compositions), which include therapeutic agents that are difficult to preserve with conventional preservatives. Additionally or alternatively, the compositions of the present invention can partially or entirely avoid conventional preservatives that, while potentially still efficacious, are also undesirable due to issues such as toxicity and/or discomfort.

Thus, sorbate and the diquaternary ammonium compound[s] may be utilized as antimicrobial preservatives for the compositions of the present invention in place of conventional, antimicrobial agents known to those skilled in the art, for example, benzalkonium chloride or polyquad-1. More specifically, the pharmaceutical compositions of the present invention may be preserved and be entirely free or free of any preservative amount of conventional antimicrobial preservative agents, such as benzalkonium chloride, benzalkonium bromide, polyquaternium-1, chlorhexidine, chlorobutanol, cetylpyridinium chloride, parabens, thimerosal, chlorine dioxide and N,N-dichlorotaurine. However, the compounds of formula (I) may also be used in combination with conventional preservative ingredients to further increase antimicrobial activity or preservative efficacy of the compositions of the present invention. Moreover, such conventional preservatives may also benefit from the lower ionic strength of the compositions as is shown in the examples below.

Therapeutic agents that benefit from delivery in the pharmaceutical compositions of the present invention are those agents that inhibit the preservative efficacy of conventional preservatives themselves and/or agents that are best delivered under conditions that inhibit the preservative efficacy of conventional preservatives. Of particular importance are agents that are significantly more soluble at lower pH values. At such lower pH values, many of the conventional preservatives, particularly polyquaternium-1, can experience reduced efficacy. However, the agents discussed herein (i.e., the agents significantly affected by ionic strength), particularly sorbate, diquaternary ammonium compound (e.g., SCDCP) or both, can retain their preservation efficacy at such lower pH values as long as the ionic strength of the overall composition is kept relatively low. Thus, in certain embodiment of the present invention, the pH of the composition of the present invention is typically less than 7.0, more typically less than 6.0 and even possibly less than 5.5 or even 5.2.

Generally, most any suitable therapeutic agents may be included in the compositions of the present invention. However, therapeutic agents, which may be included in the composition of the present invention and which exhibit higher solubility at lower pH levels include, without limitation, emadastine, olapatadine, any combination thereof or the like. Therapeutic agents of particular interest to the present invention are receptor agonists. Particularly preferred pharmaceutical compositions of the present invention include a serotonin $5HT_{1A}$ receptor agonist and, more particularly include tandospirone, which, as used herein, includes tandospirone and any pharmaceutically acceptable salt or other form of tandospirone. These particularly preferred compositions are particularly desirable for topical treatment of age related macular degeneration (AMD) and AMD related maladies such as geographic atrophy secondary to wet AMD.

Notably, the therapeutic agents that are more soluble at lower pH levels are often agents that are ionic and can significantly add to the ionic strength of the overall composition. It is possible that the therapeutic agent[s] may be responsible for at least 10%, 30% or even at least 40% of the ionic strength of the composition depending upon their degree of ionization, type of salt and counter ions.

The therapeutic agent can be present at various concentrations within the pharmaceutical composition depending upon the agent[s] present. Generally, the therapeutic agent is between about 0.01% and about 5.0% of the composition. For the $5HT_{1A}$ receptor agonist, particularly tandospirone, the concentration is typically at least 0.3%, more typically at least 0.8%, still more typically at least 1.2% and even possibly at least 1.75% and is also typically no greater than 5.0%, more typically no greater than 3.3% and even more typically no greater than 2.0% of the composition.

The compositions of the present invention may be formulated as aqueous or non-aqueous solutions, but will preferably be aqueous. Additionally, the compositions may be formulated as suspensions, gels, emulsions and other dosage forms known to those skilled in the art.

The ophthalmic, otic and nasal compositions of the present invention will be formulated so as to be compatible with the eye, ear, nose and/or contact lenses to be treated with the compositions. A preferred range of osmolality for the ophthalmic compositions of the present invention is 150 to 350 milliOsmoles per kilogram (mOsm/kg). A range of 200 to 300 mOsm/kg is particularly preferred and an osmolality of about 290 mOsm/kg is most preferred. The pH for the ophthalmic compositions of the present invention can range from about 4.5 to about 9.0 but may be preferably relatively low as detailed herein. Since, often the ophthalmic, otic and nasal formulations are required to be isotonic or near isotonic, the tonicity of the formulations can be adjusted with suitable non ionic tonicity agents including but not limited to propylene glycol, glycerin, mannitol and sorbitol.

The present invention is particularly directed to the provision of ophthalmic otic or nasal compositions that have sufficient antimicrobial activity to allow the compositions to satisfy USP preservative efficacy requirements and/or other preservative efficacy standards for aqueous pharmaceutical compositions.

The preservative efficacy standards for multi-dose pharmaceutical (e.g., ophthalmic) solutions in the U.S. and other countries/regions are set forth in the following table:

Preservative Efficacy Test ("PET") Criteria (Log Order Reduction of Microbial Inoculum Over Time

| | Bacteria | Fungi |
|---|---|---|
| USP 27 | A reduction of 1 log (90%), by day 7; 3 logs (99.9%) by day 14; and no increase after day 14 | The compositions must demonstrate over the entire test period, which means no increases of 0.5 logs or greater, relative to the initial inoculum. |
| Japan | 3 logs by 14 days; and no increase from day 14 through day 28. | No increase from initial count at 14 and 28 days |
| Ph. Eur. A[1] | A reduction of 2 logs (99%) by 6 hours; 3 logs by 24 hours; and no recovery after 28 days | A reduction of 2 logs (99%) by 7 days, and no increase thereafter |
| Ph. Eur. B | A reduction of 1 log at 24 hours; 3 logs by day 7; and no increase thereafter | A reduction of 1 log (90%) by day 14, and no increase thereafter |
| FDA/ISO 14730 | A reduction of 3 logs from initial challenge at day 14; and a reduction of 3 logs from rechallenge | No increase higher than the initial value at day 14, and no increase higher than the day 14 rechallenge count through day 28. |
| [1]There are two preservative efficacy standards in the European Pharmacopoeia "A" and "B". | | |

The standards identified above for the USP 27 are substantially identical to the requirements set forth in prior editions of the USP, particularly USP 24, USP 25 and USP 26.

Antimicrobial preservative effectiveness as set forth by the examples infra was determined using an organism challenge test according to the methods described in the United States Pharmacopeia 24 (USP) for category 1A products. Samples were inoculated with known levels of one or more of the following: gram-positive vegetative bacteria (Staphylococcus aureus ATCC 6538), gram-negative vegetative bacteria (Pseudomonas aeruginosa ATCC 9027 and Escherichia coli ATCC 8739), yeast (Candida albicans ATCC 10231) and mold (Aspergillus niger ATCC 16404). The

samples were then pulled at specified intervals to determine if the antimicrobial preservative system was capable of killing or inhibiting the propagation of organisms purposely introduced into the formulation. The rate or level of antimicrobial activity determines compliance with the USP preservative efficacy standards for the cited categories of preparations.

Preservative Standards for U.S. Category 1A Products presented as Log Reduction of Organism Population

| Time Pulls | 6 Hours | 24 Hours | 7 days | 14 days | 28 days |
|---|---|---|---|---|---|
| For Bacteria (S. aureus, P. aeruginosa, and E. coli) | | | | | |
| Ph. Eur. A (EPA) | 2.0 | 3.0 | NA | NA | NR |
| Ph. Eur. B (EPB) | NA | 1.0 | 3.0 | NI | NI |
| USP | NA | NA | 1.0 | 3.0 | NI |
| For Fungi (C. albicans and A. niger) | | | | | |
| Ph. Eur. A | NA | NA | 2.0 | NA | NI |
| Ph. Eur. B | NA | NA | NA | 1.0 | NI |
| USP | NA | NA | NI | NI | NI |
| NI = No increase at this or any following time pulls<br>NA = Time point not required for applicable standard (e.g., USP, Ph. Eur. B)<br>NR = No organisms recovered | | | | | |

As shown, the USP 27 Antimicrobial Effectiveness Test requires that compositions containing Category 1A products have sufficient anti-bacterial activity to reduce an initial inoculum of approximately $10^5$ to $10^6$ bacteria by one log (i.e., a 90% reduction in the microorganism population) over a period of seven (7) days and by three logs (i.e., a 99.9% reduction in the microorganism population) over a period of fourteen (14) days, and requires that there cannot be any increase in the microorganism population following the conclusion of the fourteen day period. Relative to fungi, the USP standards require that the compositions maintain stasis (i.e., no growth) relative to the population of the initial inoculum over the entire 28 day test period. A category 1A product is an injection, or other parenteral including emulsions, otic, sterile nasal products and ophthalmic products made with aqueous bases or vehicles.

The margin of error in calculating microorganism populations is generally accepted to be +/-0.5 logs. Accordingly, the term "stasis", as utilized herein relative to the above-discussed USP standards, means that the initial population cannot increase by more than 0.5 log orders, relative to the initial population.

## EXAMPLES

Table A below provides a listing of exemplary ingredients suitable for a reference formulation of the ophthalmic composition and a desired weight/volume percentage for those ingredients.

TABLE A

| Ingredient | Amount (% w/v) |
|---|---|
| Tandospirone (e.g. Tandospirone, Tandospirone HCl and/or Tandospirone Citrate) | 0.1-3.0 |
| Sorbic Acid | 0.2-0.6 |
| Propylene Glycol | 0.5-1.5 |
| Sodium Chloride | less than 0.25 |
| Hydrochloric Acid | q.s. to pH 5.0 -7.0 |
| Sodium Hydroxide | q.s. to pH 5.0 -7.0 |
| Purified water | Q.S. 100 |

For exemplary purposes, Table B provides a reference formulation and the ionic strength of that formulation is calculated.

TABLE B

| Ingredient | Amount (% w/v) |
|---|---|
| Tandospirone HCl | 1.5 |
| Sorbic Acid | 0.4 |
| Propylene Glycol | 0.9 |
| Sodium Chloride | 0.1 |
| Hydrochloric Acid | q.s. to pH 5.0 -7.0 |
| Sodium Hydroxide | q.s. to pH 5.0 -7.0 |
| Purified water | Q.S. 100 |

Calculated ionic strength of the formulation in Table B (with 1.5% Tandospirone as free base) is 0.07 mol/L, out of which Tandospirone hydrochloride contributes 0.04 mol/L and sodium chloride contributes about 0.03 mol/L.

Other embodiments of the present invention will be apparent to those skilled in the art from consideration of the present specification and practice of the present invention disclosed herein.

The following examples are presented to further illustrate selected embodiments of the present invention. The formulations shown in the examples were prepared using procedures that are well-known to persons of ordinary skill in the field of ophthalmic pharmaceutical compositions.

The formulations of Examples A-U are provided to illustrate the desirability of the present invention. The examples illustrate the antimicrobial activity and/or preservative efficacy of the pharmaceutical compositions of the present invention, which have low ionic strength, low pH and preservatives affected by ionic strength or any combination thereof. The examples also illustrate how certain conventional preservatives are affected by ionic strength. Percentages of ingredients in Examples A-U are weight/volume percents.

Examples A through E

Table C provides formulations A through E and data related to those formulations.

Preservative Efficacy Test results of Tandospirone Ophthalmic Formulations with Sorbic acid-Effect of Ionic Strength

TABLE C

| Component | Amount (% w/v) | | | | |
|---|---|---|---|---|---|
| | A | B | C | D | E |
| Tandospirone HCl | 1.925 | 1.925 | 1.925 | 1.925 | 1.925 |
| Propylene Glycol | - | 0.46 | 0.93 | 1.25 | 1.3 |
| Sorbic Acid | 0.2 | 0.2 | 0.2 | 0.2 | 0.1 |
| Sodium Chloride | 0.5 | 0.3 | 0.1 | - | - |
| Sodium hydroxide/ Hydrochloric Acid | q.s. to pH 5.0 | q.s. to pH 5.0 | q.s. to pH 5.0 | q.s. to pH 5.0 | q.s. to pH 5.0 |
| Purified Water | q.s. to 100% | q.s. to 100% | q.s. to 100% | q.s. to 100% | q.s. to 100% |
| PET Data ($Log_{10}$ Unit Reduction) | | | | | |
| S. aureus/ 6 h | 0.0 | 0.0 | +0.1 | +0.1 | 0.0 |
| S. aureus/ 24 h | 0.4 | 0.8 | 1.6 | 1.9 | 1.0 |
| S. aureus/ 7 d | D | D | 5.0 | 5.0 | 5.0 |
| S. aureus/ 14 d | D | D | NA | NA | NA |
| S. aureus/ 28 d | D | D | NA | NA | NA |
| P. aerugin/6 h | 5.0 | 5.0 | 5.0 | 5.0 | 4.3 |

(continued)

| PET Data ($Log_{10}$ Unit Reduction) | | | | | |
|---|---|---|---|---|---|
| P. aerugin/24 h | 5.0 | 5.0 | 5.0 | 5.0 | 5.0 |
| P. aerugin/7 d | D | D | 5.0 | 5.0 | 5.0 |
| P. aerugin/14 d | D | D | NA | NA | NA |
| P. aerugin/28 d | D | D | NA | NA | NA |
| E. coli/6 h | 0.2 | 0.2 | 0.2 | 0.2 | 0.2 |
| E. coli/24h | 0.4 | 1.0 | 2.4 | 3.1 | 1.3 |
| E. coli/7d | D | D | 5.1 | 5.1 | 5.1 |
| E. coli/14d | D | D | NA | NA | NA |
| E. coli/28d | D | D | NA | NA | NA |
| C. albican/7 d | D | D | 4.9 | 1.9 | 0.8 |
| C. albican/14 d | D | D | NA | NA | NA |
| C. albican/28 d | D | D | NA | NA | NA |
| A. niger/7 d | D | D | 5.3 | 5.3 | 4.2 |
| A. niger/14 d | D | D | NA | NA | NA |
| A. niger/28 d | D | D | NA | NA | NA |
| PET Results | Fail EPB | Fail EPB | Pass EPB[a] | Pass EPB[a] | Fail EPB |
| D: Discontinued since these formula did not meet 24 h EPB requirement.<br>NA: Not Applicable since these were PET screen (up to 7 days)<br>[a]Projected to pass EPB | | | | | |

The results in Table C above demonstrate the preservation of Tandospirone ophthalmic formulations with sorbic acid as a preservative and also demonstrate the effect of ionic strength on preservation. The examples suggest that preservative efficacy depends in part upon both the concentration of the sorbic acid present and upon the ionic strength of the composition. Thus, preservation in formulation D with 0.2% sorbic acid is projected to pass Ph. Eur. B whereas formulation E containing 0.1% sorbic acid is not projected to pass Ph. Eur. B. With the increase of ionic strength of the formulations as a result of increased ionic solutes (>0.1% sodium chloride), preservation efficacy is decreased. For example, formulations B and A demonstrate that preservative efficacy decreases as the ionic strength increases (relative to formulations D and C) due to an increase in the amount of sodium chloride concentration, when the sorbic acid concentration is fixed at 0.2%.

Examples F through K (reference examples)

Table D below illustrates the effect of Ionic Strength on Benzalkonium Chloride (BAC) and Polyquad preserved formulations (i.e., formulations F through K):

TABLE D

| Example | F | G | H | I | J | K |
|---|---|---|---|---|---|---|
| Component | Amount (% w/v) | | | | | |
| Benzalkonium Chloride | 0.002 | 0.002 | 0.002 | - | - | - |
| Polyquaternium-1 | - | - | - | 0.0005 | 0.0005 | 0.0005 |
| Dibasic Sodium Phosphate, Doidecahydrate | 0.18 | 0.18 | 0.18 | 0.18 | 0.18 | 0.18 |
| Mannitol | 4.8 | 2.7 | 0.4 | 4.8 | 2.7 | 0.4 |
| Sodium Chloride | - | 0.4 | 0.8 | - | 0.4 | 0.8 |

(continued)

| Example | F | G | H | I | J | K |
|---|---|---|---|---|---|---|
| Component | Amount (% w/v) | | | | | |
| Sodium hydroxide/Hydrochloric acid | q.s. to pH 7.0 | q.s. to pH 7.0 | q.s. to pH 7.0 | q.s. to pH 7.0 | q.s. to pH 7.0 | q.s. to pH 7.0 |
| Purified Water | q.s. to 100 | q.s. to 100 | q.s. to 100 | q.s. to 100 | q.s. to 100 | q.s. to 100 |
| PET | $Log_{10}$ Unit Reduction | | | | | |
| S. aureus/6 h | 5.0 | 3.9 | 2.4 | 5.0 | 4.1 | 2.2 |
| S. aureus/24 h | 5.0 | 4.5 | 4.0 | 5.0 | 5.0 | 4.1 |
| S. aureus/7 d | 5.0 | 5.0 | 5.0 | 5.0 | 5.0 | 5.0 |
| S. aureus/14 d | 5.0 | 5.0 | 5.0 | 5.0 | 5.0 | 5.0 |
| S. aureus/28d | 5.0 | 5.0 | 5.0 | 5.0 | 5.0 | 5.0 |
| P. aerugin/6 h | 4.8 | 4.8 | 3.1 | 5.1 | 5.1 | 3.5 |
| P. aerugin/24 h | 4.8 | 4.8 | 3.5 | 5.1 | 5.1 | 4.9 |
| P. aerugin/7 d | 4.8 | 4.8 | 0 | 5.1 | 5.1 | 5.1 |
| P. aerugin/14 d | 4.8 | 4.8 | +0.2 | 5.1 | 5.1 | 5.1 |
| P. aerugin/28d | 4.8 | 4.8 | +0.2 | 5.1 | 5.1 | 5.1 |
| E. coli/6 h | 4.9 | 4.9 | 4.9 | 5.0 | 5.0 | 4.9 |
| E. coli /24 h | 4.9 | 4.9 | 4.9 | 5.0 | 5.0 | 5.0 |
| E. coli/7 d | 4.9 | 4.9 | 4.9 | 5.0 | 5.0 | 5.0 |
| E. coli/14 d | 4.9 | 4.9 | 4.9 | 5.0 | 5.0 | 5.0 |
| E. coli/28d | 4.9 | 4.9 | 4.9 | 5.0 | 5.0 | 5.0 |
| C. albican/7 d | 4.7 | 4.7 | 4.7 | 4.8 | 4.3 | 1.9 |
| C. albican/14 d | 4.7 | 4.7 | 4.7 | 4.8 | 4.8 | 3.0 |
| C. albican/28 d | 4.7 | 4.7 | 4.7 | 4.8 | 4.8 | 4.8 |
| A. niger/7 d | 0.8 | 0 | 0 | 0.1 | 0.2 | 0.2 |
| A.niger/14 d | 0.8 | 0 | 0 | 0.1 | 0.1 | 0 |
| A. niger/28d | 1.0 | 0.6 | 0.5 | 0.1 | 0.3 | 0.1 |
| Result | USP | USP | Fail | USP | USP | USP |

As can be seen, increase of sodium ion has a negative impact on 0.002% BAC containing formulations (formulations F, G and H), particularly against S. aureus and P. aerugin. Formulation H with 0.8% sodium chloride has the highest ionic strength and failed the PET test and regrowth of P.aerugin was also observed. For Polyquad based formulations (formulations I, J, K), ionic strength appears to have an effect, particularly against S. aureus, P. aerugin and C. albican.

Examples L through P (reference examples)

Table E below illustrates the effect of Ionic Strength on zinc chloride Formulations (i.e., formulations L through P):

TABLE E

| Example | L | M | N | O | P |
|---|---|---|---|---|---|
| Component | Amount (% w/v) | | | | |
| Zinc Chloride | 0.0025 | 0.0025 | 0.0025 | 0.0025 | 0.0025 |
| Boric acid | 0.5 | 0.5 | 0.5 | 0.5 | 0.5 |
| Mannitol | - | - | - | 0.2 | - |
| Propylene Glycol | 1.5 | 1.0 | 0.5 | 1.0 | 0.1 |
| Sodium chloride | - | 0.21 | 0.42 | 0.21 | 0.6 |
| Sodium Hydroxide/ Hydrochloric acid | q.s. to pH 7.5 | q.s. to pH 7.5 | q.s. to pH 7.5 | q.s. to pH 7.5 | q.s. to pH 7.5 |
| Purified water | q.s. to 100% | q.s. to 100% | q.s. to 100% | q.s. to 100% | q.s. to 100% |
| PET | $Log_{10}$ Unit Reduction | | | | |
| S. aureus /6 h | 0.6 | 0.1 | 0.1 | 0.1 | 0.1 |
| S. aureus24 h | 2.1 | 1.1 | 0.5 | 0.6 | 0.3 |
| S. aureus/7 d | 5.1 | 5.1 | 3.7 | 4.5 | 3.5 |
| S. aureus/14 d | 5.1 | 5.1 | 5.0 | 5.1 | 5.0 |
| S. aureus/28d | 5.1 | 5.1 | 5.0 | 5.0 | 5.0 |
| P. aerugin/ 6h | 2.5 | 0.8 | 0.3 | 0.5 | 0.2 |
| P. aerugin/ 24h | 3.4 | 2.5 | 1.3 | 2.0 | 1.3 |
| P. aerugin/7d | 4.9 | 4.9 | 4.9 | 4.9 | 4.9 |
| P. aerugin /14 d | 4.9 | 4.9 | 4.9 | 4.9 | 4.9 |
| P. aerugin/28d | 4.9 | 4.9 | 4.9 | 4.9 | 4.9 |
| E. coli/ 6 h | 1.3 | 0.9 | 0.4 | 0.9 | 1.1 |
| E. coli /24 h | 1.9 | 1.3 | 1.3 | 1.2 | 1.1 |
| E. coli/ 7 d | 5.0 | 5.0 | 4.2 | 4.9 | 4.5 |
| E. coli /14 d | 5.0 | 5.0 | 4.9 | 5.0 | 4.9 |
| E. coli /28 d | 5.0 | 5.0 | 4.9 | 5.0 | 4.9 |
| C. albican/ 7 d | 0.7 | 0.4 | 0.5 | 0.5 | 0.1 |
| C. albican/ 14 d | 1.7 | 1.6 | 1.6 | 1.0 | 0.6 |
| C. albican /28d | 3.1 | 2.5 | 2.3 | 1.5 | 1.7 |
| A. niger /7 d | 0.9 | 1.5 | 1.9 | 1.8 | 2.1 |
| A. niger/14 d | 0.9 | 1.5 | 1.9 | 1.6 | 2.1 |
| A. niger/28d | 1.0 | 1.5 | 1.6 | 1.7 | 2.7 |
| RESULTS | USP | USP | USP | USP | USP |
| Osmolality | 293 | 281 | 283 | 287 | 284 |

As can be seen, in zinc chloride preserved formulations, sodium ions have an effect on preservation, especially on S. aureus and P. aerugin. With increase of sodium ions in the formulations, preservation effect was decreased. Formulation P containing 0.6% of sodium ions was worse compared to Formulation L without any sodium ions.

Examples Q through U

Table F below illustrates the effect of Ionic Strength on SCDCP Formulations (i.e., formulations Q through U):

TABLE F

| Example | Q | R | S | T | U |
|---|---|---|---|---|---|
| Component | Amount (W/V %) | | | | |
| SCDCP | 0.0025 | 0.0025 | 0.0025 | 0.0025 | 0.0025 |
| Boric acid | 0.5 | 0.5 | 0.5 | 0.5 | 0.5 |
| Propylene glycol | 1.5 | 1.5 | 1.5 | 1.4 | 0.1 |
| Calcium Chloride, dihydrate | - | 0.01 | 0.05 | 0.1 | - |
| Sodium chloride | - | - | - | - | 0.6 |
| Sodium Hydroxide/ Hydrochloric acid | q.s. to pH 7.0 | q.s. to pH 7.0 | q.s. to pH 7.0 | q.s. to pH 7.0 | q.s. to pH 7.0 |
| Purified water | q.s. to 100% | q.s. to 100% | q.s. to 100% | q.s. to 100% | q.s. to 100% |
| PET | $Log_{10}$Unit Reduction | | | | |
| S. aureus/ 6 h | 5.1 | 5.0 | 5.0 | 5.0 | 0.3 |
| S. aureus/ 24 h | 5.1 | 5.0 | 5.0 | 5.0 | 1.5 |
| S. aureus/ 7 d | 5.1 | 5.0 | 5.0 | 5.0 | 5.0 |
| S. aureus/14 d | 5.1 | 5.0 | 5.0 | 5.0 | 5.0 |
| S. aureus/ 28 d | 5.1 | 5.0 | 5.0 | 5.0 | 5.0 |
| P. aerugin/6 h | 5.0 | 0.4 | 1.5 | 0.3 | 0.3 |
| P. aerugin/24 h | 5.0 | 0.6 | 0.3 | 0.6 | 0.5 |
| P. aerugin/7 d | 5.0 | 1.0 | 0.7 | 0.4 | 0.6 |
| P. aerugin/14 d | 5.0 | 1.6 | 0.4 | 0.4 | 0.7 |
| P. aerugin/28 d | 5.0 | 2.3 | 0.6 | 0.3 | 0.7 |
| E. coli/6 h | 4.9 | 0.1 | 0.1 | 0.1 | -0.1 |
| E. coli/24h | 4.9 | 0.2 | 0.2 | 0.2 | 0.1 |
| E. coli/7d | 4.9 | 0.7 | 0.7 | 0.5 | 0.2 |
| E. coli/14d | 4.9 | 1.4 | 1.4 | 0.8 | 0.6 |
| E. coli/28d | 4.9 | 2.6 | 3.2 | 1.5 | 0.6 |
| C. albican/7 d | 4.9 | 4.9 | 4.9 | 4.8 | 0.6 |
| C. albican/14 d | 4.9 | 4.9 | 4.9 | 4.8 | 1.5 |
| C. albican/28 d | 4.9 | 4.9 | 4.9 | 4.8 | 4.2 |
| A. niger/7 d | 1.7 | 0.9 | 0.9 | 1.3 | 1.0 |
| A. niger/14 d | 1.5 | 0.7 | 1.0 | 1.8 | 0.6 |
| A. niger/28 d | 1.5 | 1.5 | 3.3 | 4.6 | 0.6 |
| RESULTS | EPA | Fail | Fail | Fail | Fail |
| Osmolality (mOsm/kg) | 304 | 279 | 287 | 284 | 283 |

As can be seen, $Ca^{++}$ has a profound effect of the preservation efficacy of SCDCP preserved formulations. This is evident against P. aerugin, E. coli and A. niger. $Na^{+}$ also exhibit a significant effect on SCDCP preserved formulations. This is evident on all five organisms.

## Claims

1. A pharmaceutical composition, comprising:

anti-microbial preservative that is significantly affected by ionic strength wherein the preservative is selected from sorbate or a non-polymeric diquaternary ammonium phosphate compound; and
water;
wherein, when the preservative is a non-polymeric diquaternary ammonium phosphate compound, the composition has an ionic strength that is less than 0.08 mol*$L^{-1}$; and
wherein, when the non-polymeric diquaternary ammonium phosphate compound is sodium coco diammonium chloride phosphate, the concentration of the sodium coco diammonium chloride phosphate in the composition is 0.0025 to 0.004 w/v%; or,
when the preservative is sorbate, the concentration of sorbate in the composition is from 0.01 to 0.2 w/v% and the ionic strength of the composition is less than 0.07 mol/L.

2. A composition as in claim 1 further comprising a therapeutic agent.

3. A composition as in claim 1 or 2 wherein the therapeutic agent is more soluble at lower pH levels.

4. A composition as in claim 2 or 3 wherein the therapeutic agent is tandospirone.

5. A composition as in claim 1, 2, 3 or 4 wherein the preservative is sorbate.

6. A composition as in claim 1, 2, 3 or 4 wherein the preservative is the diquaternary compound and the diquaternary compound includes a phosphate group.

7. A composition as in claim 1, 2, 3, 4 or 6 wherein the preservative is the diquaternary compound and the diquaternary compound is of formula I:

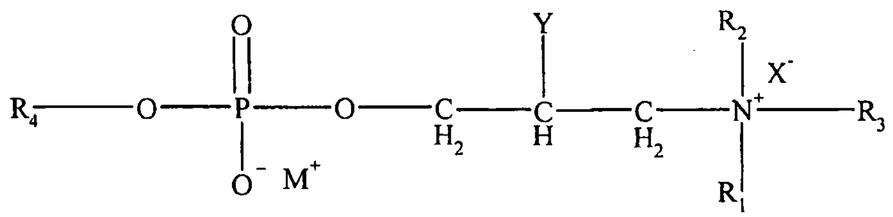

(I)

wherein:

$R_1$ and $R_3$ are ($C_1$-$C_6$)-alkyl;
$R_2$ is selected from the group consisting of hydrogen and ($C_1$-$C_{16}$)-alkyl optionally substituted by $NHC(=O)-(CH_2)_{10}CH_3$ or $NHC(=O)-(CH_2)_{12}CH_3$;
$R_4$ is selected from the group consisting of hydrogen and $CH_2CH(Y)CH_2N^+R_1R_2R_3X^-$, wherein $R_1$, $R_2$, and $R_3$, are as defined above;
X is halo;
Y is selected from the group consisting of OH, O-($C_1$-$C_{10}$)-alkyl and O-($C_1$-$C_{10}$)-alkenyl; and
M is selected from the group consisting of sodium and potassium.

8. A composition as in any of the preceding claims wherein the preservative is sodium coco diammonium chloride phosphate.

9. A composition as in any of the preceding claims wherein the pH is less than 7.0.

10. A composition as in any of the preceding claims wherein the composition is an ophthalmic, otic or nasal composition, preferably an ophthalmic composition.

11. A composition as in any of the preceding claims wherein the composition satisfies USP, Ph. Eur. A, Ph. Eur. B or any combination thereof.

12. A composition as in any of the preceding claims wherein the composition is free of any preservative effective amount of polymeric quaternary ammonium compound or benzalkonium chloride.

13. A composition as in any of the preceding claims wherein the therapeutic agent is more soluble at a pH of less than 6.0.

14. A composition as in any of the preceding claims wherein the pH is less than 6.0, preferably less than 5.5.

15. A composition as in claim 5, wherein the ionic strength of the composition is from 0.01 to 0.05 mol/L.

16. A composition as in claim 15, wherein the concentration of sorbate in the composition is less than 0.1 %.

17. A composition as in claim 16, wherein the concentration of sorbate in the composition is less than 0.03%.

18. A composition as in any of claims 1-4 and 6-14 wherein the preservative is the non-polymeric diquaternary ammonium phosphate compound, the concentration of the preservative in the composition is from 0.0025 to 0.004 w/v% and the ionic strength of the composition is from 0.01 mol/L to 0.07 mol/L.

**Patentansprüche**

1. Pharmazeutische Zusammensetzung, umfassend:

   ein antimikrobielles Konservierungsmittel, das signifikant von der Ionenstärke beeinflusst wird, wobei das Konservierungsmittel ausgewählt ist aus Sorbat oder einer nicht-polymeren Diquartärammoniumphosphatverbindung; und
   Wasser;
   wobei, wenn das Konservierungsmittel eine nicht-polymere Diquartärammoniumphosphatverbindung ist, die Zusammensetzung eine Ionenstärke hat, die geringer ist als 0,08 $mol \cdot l^{-1}$; und
   wobei, wenn die nicht-polymere Diquartärammoniumphosphatverbindung Natriumcocodiammoniumchloridphosphat ist, die Konzentration des Natriumcocodiammoniumchloridphosphats in der Zusammensetzung 0,0025 bis 0,004 % Gew./Vol. beträgt; oder, wenn das Konservierungsmittel Sorbat ist, die Konzentration des Sorbats in der Zusammensetzung 0,01 bis 0,2 % Gew./Vol. beträgt und die Ionenstärke der Zusammensetzung geringer ist als 0,07 mol/l.

2. Zusammensetzung nach Anspruch 1, ferner umfassend ein therapeutisches Mittel.

3. Zusammensetzung nach Anspruch 1 oder 2, wobei das therapeutische Mittel bei niedrigeren pH-Werten löslicher ist.

4. Zusammensetzung nach Anspruch 2 oder 3, wobei das therapeutische Mittel Tandospiron ist.

5. Zusammensetzung nach Anspruch 1, 2, 3 oder 4, wobei das Konservierungsmittel Sorbat ist.

6. Zusammensetzung nach Anspruch 1, 2, 3 oder 4, wobei das Konservierungsmittel die Diquartärverbindung ist und die Diquartärverbindung eine Phosphatgruppe umfasst.

7. Zusammensetzung nach Anspruch 1, 2, 3, 4 oder 6, wobei das Konservierungsmittel die Diquartärverbindung ist und die Diquartärverbindung die Formel I aufweist:

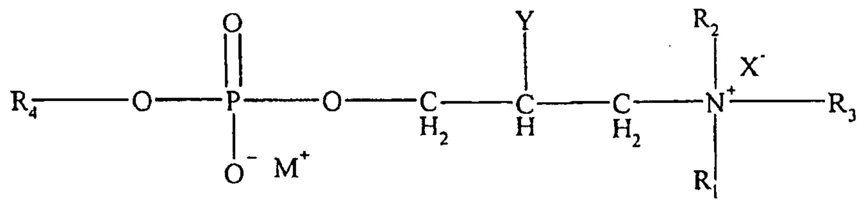

(I) ,

wobei:

$R_1$ und $R_3$ ($C_1$-$C_6$)-Alkyl sind;
$R_2$ ausgewählt ist aus der Gruppe, bestehend aus Wasserstoff und ($C_1$-$C_{16}$)-Alkyl, gegebenenfalls substituiert durch NHC(=O)-$(CH_2)_{10}CH_3$ oder NHC(=O)-$(CH_2)_{12}CH_3$;
$R_4$ ausgewählt ist aus der Gruppe, bestehend aus Wasserstoff und $CH_2CH(Y)CH_2N^+R_1R_2R_3X^-$, wobei $R_1$, $R_2$ und $R_3$ wie oben definiert sind;
X Halogen ist;
Y ausgewählt ist aus der Gruppe, bestehend aus OH, O-($C_1$-$C_{10}$)-Alkyl und O-($C_1$-$C_{10}$)-Alkenyl; und
M ausgewählt ist aus der Gruppe, bestehend aus Natrium und Kalium.

**8.** Zusammensetzung nach einem der vorstehenden Ansprüche, wobei das Konservienmgsmittel Natriumcocodiammoniumchloridphosphat ist.

**9.** Zusammensetzung nach einem der vorstehenden Ansprüche, wobei der pH geringer ist als 7,0.

**10.** Zusammensetzung nach einem der vorstehenden Ansprüche, wobei die Zusammensetzung eine ophthalmische Zusammensetzung, eine Zusammensetzung für das Ohr oder eine nasale Zusammensetzung, bevorzugt eine ophthalmische Zusammensetzung ist.

**11.** Zusammensetzung nach einem der vorstehenden Ansprüche, wobei die Zusammensetzung USP, Ph. Eur. A, Ph. Eur. B oder einer Kombination davon genügt.

**12.** Zusammensetzung nach einem der vorstehenden Ansprüche, wobei die Zusammensetzung frei ist von konservierenden wirksamen Mengen einer polymeren Quartärammoniumverbindung oder Benzalkoniumchlorid.

**13.** Zusammensetzung nach einem der vorstehenden Ansprüche, wobei das therapeutische Mittel bei einem pH von weniger als 6,0 löslicher ist.

**14.** Zusammensetzung nach einem der vorstehenden Ansprüche, wobei der pH niedriger ist als 6,0, bevorzugt niedriger als 5,5.

**15.** Zusammensetzung nach Anspruch 5, wobei die Ionenstärke der Zusammensetzung 0,01 bis 0,05 mol/l beträgt.

**16.** Zusammensetzung nach Anspruch 15, wobei die Konzentration an Sorbat in der Zusammensetzung geringer ist als 0,1 %.

**17.** Zusammensetzung nach Anspruch 16, wobei die Konzentration an Sorbat in der Zusammensetzung geringer ist als 0,03 %.

**18.** Zusammensetzung nach einem der Ansprüche 1 bis 4 und 6 bis 14, wobei das Konservierungsmittel die nichtpolymere Diquartärammoniumphosphatverbindung ist, die Konzentration des Konservierungsmittels in der Zusammensetzung 0,0025 bis 0,004 % Gew./Vol. beträgt und die Ionenstärke der Zusammensetzung 0,01 mol/l bis 0,07 mol/l beträgt.

## Revendications

**1.** Composition pharmaceutique, comprenant :

un agent conservateur anti-microbien significativement affecté par une force ionique dans laquelle l'agent conservateur est choisi parmi le sorbate ou un composé non-polymérique ammonium diquaternaire phosphate; et de l'eau ;
dans laquelle, lorsque l'agent conservateur est un composé non-polymérique ammonium diquaternaire phosphate, la composition a une force ionique inférieure à 0,08 mol*$L^{-1}$ ; et
dans laquelle, lorsque le composé non-polymérique ammonium diquaternaire phosphate est le chlorure de diammonium sodium coco phosphate, la concentration en chlorure de diammonium sodium coco phosphate dans la composition est de 0,0025 à 0,004 % en poids/volume ; ou, lorsque l'agent conservateur est le sorbate, la concentration en sorbate dans la composition est de 0,01 à 0,2 % en poids/volume et la force ionique de la composition est inférieure à 0,07 mol/L.

**2.** Composition selon la revendication 1 comprenant en outre un agent thérapeutique.

**3.** Composition selon la revendication 1 ou 2 dans laquelle l'agent thérapeutique est plus soluble à des niveaux de pH plus faibles.

**4.** Composition selon la revendication 2 ou 3 dans laquelle l'agent thérapeutique est la tandospirone.

**5.** Composition selon la revendication 1, 2, 3 ou 4 dans laquelle l'agent conservateur est le sorbate.

**6.** Composition selon la revendication 1, 2, 3 ou 4 dans laquelle l'agent conservateur est le composé diquaternaire et le composé diquaternaire comprend un groupe phosphate.

**7.** Composition selon la revendication 1, 2, 3, 4 ou 6 dans laquelle l'agent conservateur est le composé diquaternaire et le composé diquaternaire est de formule 1 :

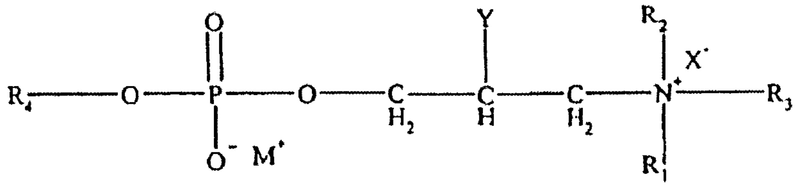

(I)

dans laquelle :

$R_1$ et $R_3$ sont ($C_1$-$C_6$)-alkyle ;
$R_2$ est choisi parmi le groupe consistant en l'hydrogène et ($C_1$-$C_{16}$)-alkyle optionnellement substitué par $NHC(=O)-(CH_2)_{10}CH_3$ ou $NHC(=O)-(CH_2)_{12}CH_3$ ;
$R_4$ est choisi parmi le groupe consistant en l'hydrogène et $CH_2CH(Y)CH_2N^+R_1R_2R_3X^-$, dans laquelle $R_1$, $R_2$, et $R_3$ sont tels que définis ci-dessus ;
X est un halogène ;
Y est choisi parmi le groupe consistant en OH, O-($C_1$-$C_{10}$)-alkyle et O-($C_1$-$C_{10}$)-alkènyle ; et
M est choisi parmi le groupe consistant en sodium et potassium.

**8.** Composition selon l'une quelconque des revendications précédentes dans laquelle l'agent conservateur est le chlorure de diammonium sodium coco phosphate.

**9.** Composition selon l'une quelconque des revendications précédentes dans laquelle le pH est inférieur à 7,0.

**10.** Composition selon l'une quelconque des revendications précédentes dans laquelle la composition est une composition ophtalmique, otique ou nasale, de préférence une composition ophtalmique.

**11.** Composition selon l'une quelconque des revendications précédentes dans laquelle la composition satisfait USP, Ph. Eur. A, Ph. Eur. B ou une combinaison de ceux-ci.

**12.** Composition selon l'une quelconque des revendications précédentes dans laquelle la composition est libre de toute quantité efficace du point de vue de la conservation de composé polymérique ammonium quaternaire ou le chlorure de benzalkonium.

**13.** Composition selon l'une quelconque des revendications précédentes dans laquelle l'agent thérapeutique est plus soluble à un pH inférieur à 6,0.

**14.** Composition selon l'une quelconque des revendications précédentes dans laquelle le pH est inférieur à 6,0, de préférence inférieur à 5,5.

**15.** Composition selon la revendication 5, dans laquelle la force ionique de la composition est de 0,01 à 0,05 mol/L.

**16.** Composition selon la revendication 15, dans laquelle la concentration en sorbate dans la composition est inférieure à 0,1%.

**17.** Composition selon la revendication 16, dans laquelle la concentration en sorbate dans la composition est inférieure à 0,03%.

**18.** Composition selon les revendications 1-4 et 6-14 dans laquelle l'agent conservateur est le composé non-polymérique ammonium diquaternaire phosphate, la concentration de l'agent conservateur dans la composition est de 0,0025 à 0,004 % en poids/volume et la force ionique de la composition est de 0,01 mol/L à 0,07 mol/L.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5286719 A
- US 5648348 A
- US 5650402 A
- US 12219708 A